(19) Europäisches Patentamt
European Patent Office
Office européen des brevets

(11) Veröffentlichungsnummer: **0 116 932**
**A1**

# (12) EUROPÄISCHE PATENTANMELDUNG

(21) Anmeldenummer: **84101466.5**

(22) Anmeldetag: **13.02.84**

(51) Int. Cl.³: **C 07 D 333/38**
A 01 N 47/36

(30) Priorität: **19.02.83 DE 3305866**

(43) Veröffentlichungstag der Anmeldung:
**29.08.84 Patentblatt 84/35**

(84) Benannte Vertragsstaaten:
**DE FR GB IT**

(71) Anmelder: **BASF Aktiengesellschaft**
**Carl-Bosch-Strasse 38**
**D-6700 Ludwigshafen(DE)**

(72) Erfinder: **Acker, Rolf-Dieter, Dr.**
**Tuchbleiche 8**
**D-6906 Leimen(DE)**

(72) Erfinder: **Rossy, Phillip A., Dr.**
**39, Forest Drive**
**Hillsdale N.J. 07642(US)**

(72) Erfinder: **Wuerzer, Bruno, Dipl.-Landwirt, Dr.**
**Ruedigerstrasse 13**
**D-6701 Otterstadt(DE)**

(54) **Thiophen-carbonester, Verfahren zu ihrer Herstellung und ihre Verwendung zur Bekämpfung unerwünschten Pflanzenwuchses.**

(57) Die Erfindung betrifft Thiophen-carbonester der Formel

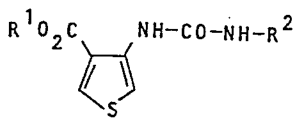

in der

$R^1$ Wasserstoff, Alkyl, Alkenyl, Alkinyl, Halogenalkyl, Alkoxyalkyl, Alkythioalkyl, Cycloalkyl, gegebenenfalls substituiertes Phenyl oder Benzyl und

$R^2$ Alkyl, Alkenyl, Alkinyl, gegebenenfalls substituiertes Phenylalkyl, Halogenalkyl, Alkoxyalkyl, Alkylthioalkyl, Alkylaminoalkyl, Dialkylaminoalkyl, Cycloalkyl oder gegebenenfalls substituiertes Phenyl bedeuten,

Verfahren zur ihrer Herstellung und ihre Verwendung zur Bekämpfung unerwünschten Pflanzenwuchses.

EP 0 116 932 A1

Croydon Printing Company Ltd.

Thiophen-carbonester, Verfahren zu ihrer Herstellung und ihre Verwendung zur Bekämpfung unerwünschten Pflanzenwuchses

Die Erfindung betrifft Thiophen-carbonester, Verfahren zu ihrer Herstellung, Herbizide, die diese Verbindungen als Wirkstoffe enthalten, sowie ein Verfahren zur Bekämpfung unerwünschten Pflanzenwuchses mit diesen Wirkstoffen.

Es wurde gefunden, daß Thiophen-carbonester der Formel

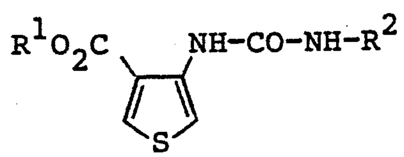
(I),

in der

$R^1$ Wasserstoff, $C_1$-$C_{10}$-Alkyl, $C_2$-$C_{10}$-Alkenyl, $C_2$-$C_{10}$-Alkinyl, $C_1$-$C_{10}$-Halogenalkyl, $C_2$-$C_{10}$-Alkoxyalkyl, $C_2$-$C_{10}$-Alkylthioalkyl, $C_3$-$C_7$-Cycloalkyl, gegebenenfalls durch Halogen oder $C_1$-$C_4$-Alkyl substituiertes Phenyl oder gegebenenfalls durch Halogen substituiertes Benzyl und

$R^2$ $C_1$-$C_{10}$-Alkyl, $C_2$-$C_{10}$-Alkenyl, $C_2$-$C_{10}$-Alkinyl, gegebenenfalls durch Halogen substituiertes $C_7$-$C_{10}$-Phenylalkyl, $C_1$-$C_{10}$-Halogenalkyl, $C_2$-$C_{10}$-Alkoxyalkyl, $C_2$-$C_{10}$-Alkylthioalkyl, durch Alkylamino oder Dialkylamino mit 1 bis 4 C-Atomen in einer Alkylgruppe substituiertes $C_1$-$C_{10}$-Alkyl, $C_3$-$C_7$-Cycloalkyl oder gegebenenfalls durch Halogen oder $C_1$-$C_4$-Alkyl substituiertes Phenyl bedeuten,

herbizid wirksam sind.

$R^1$ und $R^2$ in Formel I bedeuten unverzweigtes oder verzweigtes $C_1$-$C_{10}$-Alkyl, vorzugsweise $C_1$-$C_4$-Alkyl, unverzweigtes oder verzweigtes $C_2$-$C_{10}$-Alkenyl, vorzugsweise $C_3$-$C_4$-Alkenyl, unverzweigtes oder verzweigtes $C_2$-$C_{10}$-Alkinyl, vorzugsweise $C_3$-$C_4$-Alkinyl, gegebenenfalls durch Halogen substituiertes $C_7$-$C_{10}$-Phenylalkyl, vorzugsweise $C_8$-$C_9$-Phenylalkyl, unverzweigtes oder verzweigtes $C_1$-$C_{10}$-Halogenalkyl, vorzugsweise $C_1$-$C_4$-Halogenalkyl, unverzweigtes oder verzweigtes $C_2$-$C_{10}$-Alkoxyalkyl oder $C_2$-$C_{10}$-Alkylthioalkyl, vorzugsweise $C_2$-$C_4$-Alkoxyalkyl oder $C_2$-$C_4$-Alkylthioalkyl, durch Alkylamino oder Dialkylamino mit 1 bis 4 C-Atomen in einer Alkylgruppe substituiertes $C_1$-$C_{10}$-Alkyl, vorzugsweise $C_1$-$C_4$-Alkyl, oder $C_3$-$C_7$-Cycloalkyl, vorzugsweise $C_5$-$C_6$-Cycloalkyl, beispielsweise Ethyl, n-Propyl, Isopropyl, n-Butyl, Isobutyl, sec-Butyl, tert.-Butyl, n-Pentyl, n-Hexyl, Pentyl-3, 1,2-Dimethyl-n-propyl,

H/uw

1,3-Dimethyl-n-butyl, 1-Ethyl-2-methyl-n-propyl, 1,2,2-Trimethyl-n--propyl, 1,2-Dimethyl-n-hexyl, tert.-Amyl, Vinyl, Allyl, Methallyl, Crotyl, 2-Ethyl-hexen-2-yl, Hexen-5-yl, 2-Methyl-buten-2-yl, 2-Methyl--buten-1-yl-3, Butin-1-yl-3, Butin-2-yl, Buten-1-yl-3, Propargyl, 2-Methyl-buten-2-yl-4, 2-Methyl-buten-2-yl-4, 3-Methyl-buten-1-yl-3, 2-Phenylethyl, Benzyl, am Phenylring durch Halogen, wie Fluor, Chlor, Brom, Jod, substituiertes Benzyl, wie 2,6-Dichlorbenzyl, 2-Chlor-6-fluor--benzyl, 2,6-Difluorbenzyl, 3-Phenyl-n-propyl, 2-Chlorethyl, 2-Chlor-n--propyl, 3-Chlor-n-propyl, 2-Chlor-isopropyl, 1-Chlormethyl-n-propyl; 2-Chlorbutyl-3, 2-Chlor-2-methyl-n-propyl, 2-Fluorbutyl-3, 2-Fluor-2--methyl-n-propyl, 2-Fluor-isopropyl, Chlor-tert-butyl, 2,2,2-Trifluor--ethyl, Methoxyethyl, Ethoxyethyl, 3-Methoxy-n-propyl, Methoxyisopropyl, 3-Methoxy-n-butyl, 1-Methoxy-butyl-2, Ethoxy-tert-butyl, Methoxy-tert--butyl, 2-Methoxy-butyl, 4-Methoxy-n-butyl, Methylmercapto-ethyl, Ethylmercapto-ethyl, 3-Methylmercapto-n-propyl, 3-Methylmercapto-n-butyl, 1-Methylmercapto-butyl-2, Methylmercapto-tert-butyl, 2-Methylmercapto--n-butyl, 2-Dimethylamino-ethyl, 2-Methylamino-ethyl, 2-Diethylamino--ethyl, Dimethylaminomethyl, Dimethylaminoethyl, Cyclopropyl, Cyclopentyl, Cyclohexyl, Cycloheptyl.

$R^1$ und $R^2$ können auch einen gegebenenfalls durch Halogen, wie Fluor, Chlor, Brom, Iod, oder $C_1$-$C_4$-Alkyl substituierten Phenylrest, wie Phenyl, 4-Chlorphenyl, 2,4-Dichlorphenyl, 4-Isopropylphenyl, 4-tert.-Butylphenyl, bedeuten.

Bevorzugte Thiophen-carbonester sind Verbindungen der Formel I, wobei $R^1$ $C_1$-$C_4$-Alkyl, insbesondere Methyl, und $R^2$ $C_1$-$C_4$-Alkyl oder $C_5$-$C_6$-Cycloalkyl bedeuten.

Man erhält die Thiophen-carbonester der Formel I

a) durch Umsetzung von Dihydrothiophencarbonestern der Formel

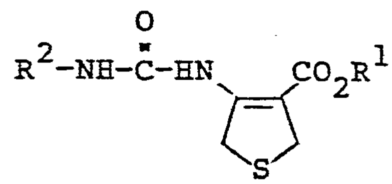 (II),

in der $R^1$ und $R^2$ die obengenannten Bedeutungen haben, mit Dehydrierungsmitteln, wie Sulfurylchlorid,

oder

b) durch Umsetzung von Aminoverbindungen der Formel

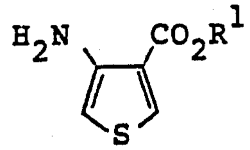

(III),

in der $R^1$ die obengenannten Bedeutungen hat, oder ihrer Salze mit einem Isocyanat der Formel

$R^2$-NCO (IV),

in der $R^2$ die obengenannten Bedeutungen hat.

Die Verfahrensvariante a) wird bei einer Temperatur im Bereich zwischen 0 und 150°C, vorzugsweise 20 und 60°C, gegebenenfalls unter Zusatz eines inerten organischen Lösungsmittels durchgeführt.

Geeignete Dehydrierungsmittel sind beispielsweise Sulfurylchlorid und Chloranil.

Zur Erhöhung der Ausbeute kann das entstehende Wasser azeotrop abdestilliert werden. Verbindung II kann in einem Überschuß oder Unterschuß von bis zu 25 Mol%, bezogen auf das Dehydrierungsmittel, eingesetzt werden.

Die Dihydrothiophen-carbonester der Formel II lassen sich beispielsweise durch Umsetzung von Ketoestern der Formel

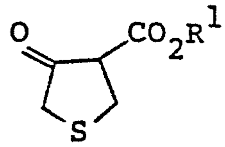

(V),

in der
$R^1$ die obengenannten Bedeutungen hat, mit Harnstoffen der Formel

$$R^2\text{-NH-}\overset{O}{\overset{\|}{C}}\text{-NH}_2 \qquad \text{(VI)},$$

in der
$R^2$ die obengenannten Bedeutungen hat, herstellen.

Die Umsetzung wird bei einer Temperatur im Bereich zwischen 0 und 150°C, vorzugsweise 50 und 120°C, gegebenenfalls unter Zusatz eines inerten organischen Lösungsmittels durchgeführt. Zweckmäßigerweise wird dem

Reaktionsgemisch ein Kondensationsmittel zugesetzt, beispielsweise p-Toluolsulfonsäure, Phosphorsäure, Polyphosphorsäure oder Schwefelsäure. Die Menge an Kondensationsmittel beträgt 0,1 bis 20 Mol%, bezogen auf Verbindung V.

Zur Erhöhung der Ausbeute kann das entstehende Wasser azeotrop abdestilliert werden. Verbindung V kann in einem Überschuß oder Unterschuß von bis zu 25 Mol%, bezogen auf Verbindung VI, eingesetzt werden.

Ketoester der Formel V, in der $R^1$ Methyl bedeutet, sind bekannt (J. Org. Chem. 45, 617 (1980)). Ketoester der Formel V, in der $R^1$ die für Formel I genannten Bedeutungen, mit Ausnahme von Methyl und Wasserstoff, hat, werden durch Umesterung von $C_1$-$C_3$-Alkylestern der Formel V mit Hydroxylverbindungen der Formel $R^1OH$, in der $R^1$ die für Formel I genannten Bedeutungen, mit Ausnahme von Methyl und Wasserstoff, hat, erhalten.

Bei dieser Reaktion werden zweckmäßigerweise basische oder saure Katalysatoren in Mengen von 0,1 bis 20 Mol.%, bezogen auf Verbindung V, zugesetzt.

Geeignete saure Katalysatoren sind beispielsweise anorganische Säuren, wie Salzsäure, Schwefelsäure, Phosphorsäure, Polyphosphorsäure, oder auch aromatische Carbonsäuren oder Sulfonsäuren, insbesondere p-Toluolsulfonsäure. Als basische Katalysatoren kommen tertiäre Amine, Erdalkaliverbindungen, Ammoniumverbindungen und Alkaliverbindungen sowie entsprechende Gemische in Betracht. Auch Zinkverbindungen können verwendet werden. Beispiele hierfür sind: Kaliumhydroxid, Natriumhydroxid, Kaliumcarbonat, Natriumcarbonat, Lithiumhydroxid, Lithiumcarbonat, Natriumhydrogencarbonat, Kaliumhydrogencarbonat, Calciumhydroxid, Calciumoxid, Bariumoxid, Magnesiumhydroxid, Magnesiumoxid, Bariumhydroxid, Calciumcarbonat, Magnesiumcarbonat, Magnesiumhydrogencarbonat, Magnesiumacetat, Zinkhydroxid, Zinkoxid, Zinkcarbonat, Zinkacetat, Natriumformiat, Natriumacetat, Trimethylamin, Triethylamin, Tripropylamin, Triisopropylamin, Tributylamin, Triisobutylamin, Tri-sec-butylamin, Tri-tert.-butylamin, Tribenzylamin, Tricyclohexylamin, Triamylamin, Diisopropylethylamin, Trihexylamin, N,N-Dimethylanilin, N,N-Diethylanilin, N,N-Dipropylanilin, N,N-Dimethyltoluidin, N,N-Diethyltoluidin, N,N-Dipropyltoluidin, N,N-Dimethyl-p-aminopyridin, N,N-Diethyl-p-aminopyridin, N,N-Dipropyl-p-aminopyridin, N-Methylpyrrolidon, N-Ethylpyrrolidon, N-Methylpiperidin, N-Ethylpiperidin, N-Methylpyrrolidin, N-Ethylpyrrolidin, N-Methylimidazol, N-Ethylimidazol, N-Methylpyrrol, N-Ethylpyrrol, N-Methylmorpholin, N-Ethylmorpholin, N-Methylhexamethylenimin, N-Ethylhexamethylenimin, Pyridin, Chinolin, alpha-Picolin, beta-Picolin, gamma-Picolin, Isochinolin, Pyrimidin, Acridin, N,N,N',N'-Tetramethylethylendiamin, N,N,N',N'-Tetraethylethylendiamin, Chinoxalin, Chinazolin, N-Propyldiisopropylamin, N,N'-Dimethylcyclohexylamin, 2,6-Lutidin, 2,4-Lutidin, Trifurylamin, Triethylendiamin.

Außer den vorgenannten anorganischen Basen kommen außerdem z.B. Natriumpropionat, Natriumbutyrat, Natriumisobutyrat, Kaliumformiat, Kaliumacetat, Kaliumpropionat, Kaliumbutyrat, Kaliumisobutyrat, Natriummethylat, Natriumethylat, Natriumpropylat, Natriumisopropylat, Natriumbutylat, Natriumisobutylat, Natrium-sec-butylat, Natrium-tert.-butylat, Natriumethylenglykolat, Natriumpropylen-(1.2)-glykolat, Natriumpropylen-(1,3)-glykolat, Natriumdiethylenglykolat, Natriumtriethylenglykolat, Natriumdipropylen-(1,2)-glykolat, Kaliummethylat, Kaliumethylat, Kalium-n-propylat, Kaliumisopropylat, Kalium-n-butylat, Kalium-isobutylat, Kalium-sec-butylat, Kalium-tert.-butylat, Kaliummethylenglykolat, Kaliumpropylen-(1,2)-glykolat, Kaliumpropylen-(1,3)-glykolat, Kaliumdiethylenglykolat, Kaliumtriethylenglykolat, Kaliumdipropylen-(1,2)-glykolat in Betracht.

Die Herstellung eines Dihydrothiophen-carbonesters der Formel II wird durch folgendes Beispiel erläutert:

15,1 Gew.-Teile 3-Keto-1,5-dihydro-thiophen-4-carbonsäuremethylester, 14,2 Gew.-Teile Cyclohexylharnstoff und 0,5 Gew.-Teile p-Toluolsulfonsäure werden in 100 Gew.-Teilen Xylol 4 Stunden unter Rückfluß bei Verwendung eines Wasserabscheiders gekocht. Nach dem Abkühlen wird der Rückstand abgesaugt und aus Toluol umkristallisiert. Man erhält 20,3 Gew.-Teile N-Cyclohexyl-N'-(3-methoxycarbonyl-2,5-dihydro-thien-4-yl)-harnstoff vom Fp. 154 bis 155°C.

Entsprechend können beispielsweise folgende Dihydrothiophen-carbonester der Formel II erhalten werden.

| $R^1$ | $R^2$ | Fp [°C] |
|---|---|---|
| $CH_3$ | $CH_3$ | 203-212 |
| $CH_3$ | $C_2H_5$ | 118-120 |
| $CH_3$ | $n-C_3H_7$ | 160-161 |
| $CH_3$ | $i-C_3H_7$ | 123-125 |
| $CH_3$ | $n-C_4H_9$ | 135-137 |
| $CH_3$ | Cyclohexyl | 154-155 |
| $CH_3$ | Phenyl | 168-171 |
| $CH_3$ | 4-Chlorphenyl | 184-187 |
| $CH_3$ | 3-Chlorphenyl | 183-185 |

| $R^1$ | $R^2$ | Fp [°C] |
|---|---|---|
| $CH_3$ | $ClCH_2CH_2$ | 133-137 |
| $CH_3$ | $CH_3CH(Cl)CH_2$ | 136-139 |
| $C_2H_5$ | $CH_3$ | 154-157 |
| $i\text{-}C_3H_7$ | $CH_3$ | 156-159 |
| $CH_3$ | 2-Phenylethyl | 117-119 |
| $n\text{-}C_4H_9$ | $n\text{-}C_4H_9$ | 100-103 |

Die Verfahrensvariante b) wird mit ungefähr stöchiometrischen Substanzmengen, d.h. in einem Mengenverhältnis von etwa 0,8 bis 1,2 Mol Verbindung III zu Verbindung IV, gegebenenfalls in Gegenwart eines inerten organischen Lösungsmittels bei einer Temperatur von -20 bis +50°C durchgeführt. Falls Verbindung III als Salz vorliegt, kann eine Base zugesetzt werden. Es kann dann entweder das freie Amin isoliert werden, oder es werden Verbindungen der Formel IV direkt zugegeben. Nach dem Einengen der Lösung reinigt man die Verbindungen der Formel I durch Umkristallisation oder Chromatographie.

Geeignete Basen sind tertiäre Amine, Erdalkaliverbindungen, Ammoniumverbindungen und Alkaliverbindungen sowie entsprechende Gemische in Betracht. Auch Zinkverbindungen können verwendet werden. Beispiele hierfür sind: Kaliumhydroxid, Natriumhydroxid, Kaliumcarbonat, Natriumcarbonat, Lithiumhydroxid, Lithiumcarbonat, Natriumhydrogencarbonat, Kaliumhydrogencarbonat, Calciumhydroxid, Calciumoxid, Bariumoxid, Magnesiumhydroxid, Magnesiumoxid, Bariumhydroxid, Calciumcarbonat, Magnesiumcarbonat, Magnesiumhydrogencarbonat, Magnesiumacetat, Zinkhydroxid, Zinkoxid, Zinkcarbonat, Zinkacetat, Natriumformiat, Natriumacetat, Trimethylamin, Triethylamin, Tripropylamin, Triisopropylamin, Tributylamin, Triisobutylamin, Tri-sec-butylamin, Tri-tert.-butylamin, Tribenzylamin, Tricyclohexylamin, Triamylamin, Diisopropylethylamin, Trihexylamin, N,N-Dimethylanilin, N,N-Diethylanilin, N,N-Dipropylanilin, N,N-Dimethyltoluidin, N,N-Diethyltoluidin, N,N-Dipropyltoluidin, N,N-Dimethyl-p-aminopyridin, N,N-Diethyl-p-aminopyridin, N,N-Dipropyl-p-aminopyridin, N-Methylpyrrolidon, N-Ethylpyrrolidon, N-Methylpiperidin, N-Ethylpiperidin, N-Methylpyrrolidin, N-Ethylpyrrolidin, N-Methylimidazol, N-Ethylimidazol, N-Methylpyrrol, N-Ethylpyrrol, N-Methylmorpholin, N-Ethylmorpholin, N-Methylhexamethylenimin, N-Ethylhexamethylenimin, Pyridin, Chinolin, alpha-Picolin, beta-Picolin, gamma-Picolin, Isochinolin, Pyrimidin, Acridin, N,N,N',N'-Tetramethylethylendiamin, N,N,N',N'-Tetraethylethylendiamin, Chinoxalin, Chinazolin, N-Propyldiisopropylamin, N,N'-Dimethylcyclohexylamin, 2,6-Lutidin, 2,4-Lutidin, Trifurylamin, Triethylendiamin.

Außer den vorgenannten anorganischen Basen kommen außerdem z.B. Natriumpropionat, Natriumbutyrat, Natriumisobutyrat, Kaliumformiat, Kaliumacetat, Kaliumpropionat, Kaliumbutyrat, Kaliumisobutyrat, Natriummethylat, Natriumethylat, Natriumpropylat, Natriumisopropylat, Natriumbutylat, Natriumisobutylat, Natrium-sec-butylat, Natrium-tert.-butylat, Natriumethylenglykolat, Natriumpropylen-(1,2)-glykolat, Natriumpropylen-(1,3)-glykolat, Natriumdiethylenglykolat, Natriumtriethylenglykolat, Natriumdipropylen-(1,2)-glykolat, Kaliummethylat, Kaliumethylat, Kalium-n-propylat, Kaliumisopropylat, Kalium-n-butylat, Kalium-isobutylat, Kalium-sec-butylat, Kalium-tert.-butylat, Kaliummethylenglykolat, Kaliumpropylen-(1,2)-glykolat, Kaliumpropylen-(1,3)-glykolat, Kaliumdiethylenglykolat, Kaliumtriethylenglykolat, Kaliumdipropylen-(1,2)-glykolat in Betracht.

Als Lösungsmittel kommen für beide Verfahrensvarianten a) und b) sowie für das Verfahren zur Herstellung der Dihydrothiophen-carbonester der Formel II z.B. Halogenkohlenwasserstoffe, insbesondere Chlorkohlenwasserstoffe, z.B. Tetrachlorethylen, 1,1,2,2- oder 1,1,1,2-Tetrachlorethan, Dichlorpropan, Methylenchlorid, Dichlorbutan, Chloroform, Chlornaphthalin, Dichlornaphthalin, Tetrachlorkohlenstoff, 1,1,1- oder 1,1,2-Trichlorethan, Trichlorethylen, Pentachlorethan, o-, m-, p-Difluorbenzol, 1,2-Dichlorethan, 1,1-Dichlorethan, 1,2-cis-Dichlorethylen, Chlorbenzol, Fluorbenzol, Brombenzol, Jodbenzol, o-, p- und m-Dichlorbenzol, o-, p-, m-Dibrombenzol, o-, m-, p-Chlortoluol, 1,2,4-Trichlorbenzol; Ether, z.B. Ethylpropylether, Methyl-tert.-butylether, n-Butylethylether, Di-n-butylether, Diisobutylether, Diisoamylether, Diisopropylether, Anisol, Phenetol, Cyclohexylmethylether, Diethylether, Ethylenglykoldimethylether, Tetrahydrofuran, Dioxan, Thioanisol, beta,beta'-Dichlordiethylether; Nitrokohlenwasserstoffe, wie Nitromethan, Nitroethan, Nitrobenzol, o-, m-, p-Chlornitrobenzol, o-Nitrotoluol; Nitrile, wie Acetonitril, Butyronitril, Isobutyronitril, Benzonitril, m-Chlorbenzonitril; aliphatische, cycloaliphatische oder aromatische Kohlenwasserstoffe, z.B. Heptan, Pinan, Nonan, o-, m-, p-Cymol, Benzinfraktionen innerhalb eines Siedepunktintervalls von 70 bis 190°C, Cyclohexan, Methylcyclohexan, Dekalin, Petrolether, Hexan, Ligroin, 2,2,4-Trimethylpentan, 2,2,3-Trimethylpentan, 2,3,3-Trimethylpentan, Octan, Toluol, o-, m-, p-Xylol, Tetralin; Ester, z.B. Ethylacetat, Acetessigester, Isobutylacetat; Amide, z.B. Formamid, Methylformamid, Dimethylformamid; Ketone, z.B. Aceton, Methylethylketon; und entsprechende Gemische in Betracht. Zweckmäßigerweise verwendet man das Lösungsmittel in einer Menge von 100 bis 2000 Gew.%, vorzugsweise von 200 bis 700 Gew.%, bezogen auf Ausgangsstoff II bzw. IV.

Beide Verfahren können kontinuierlich oder diskontinuierlich, drucklos oder unter Druck, durchgeführt werden; der Einfachheit halber wird Atmosphärendruck bevorzugt.

Beispiel 1

9,3 Gew.-Teile 3-Amino-4-methoxycarbonyl-thiophen-hydrochlorid, 6,0 Gew.-Teile Triethylamin, 7,4 Gew.-Teile Cyclohexylisocyanat und 30 Gew.-Teile Acetonitril werden zusammengegeben und 3 Stunden bei 25°C gerührt. Nach dem Einengen wird der Rückstand mit Wasser gewaschen und aus Toluol umkristallisiert. Man erhält 4,5 Gew.-Teile N-Cyclohexyl--N'-(3-methoxycarbonyl-thien-4-yl)-harnstoff vom Fp. 108 bis 114°C.

Beispiel 2

9,0 Gew.-Teile N-(n-Propyl)-N'-(3-isobutoxycarbonyl-2,5-dihydro-thien-4--yl)-harnstoff werden in 55 Teilen trockenem Chloroform vorgelegt. 2,6 Teile Sulfurylchlorid werden bei 30 bis 40°C zugetropft. Die Mischung wird 7 Stunden bei 40°C gehalten. Nach dem Abdestillieren des Lösungsmittels bleibt ein viskoses Öl zurück, das durch Verteilung in Wasser/Methylenchlorid gereinigt werden kann. Man erhält 7,6 Teile N-(n-Propyl)-N'-(3-isobutoxycarbonyl-thien-4-yl)-harnstoff.

$^1$H-NMR: $\delta$ = 7,7 und 8,0 (2 Dubletts, 2 Thiophen-H)

Entsprechend können beispielsweise folgende Thiophen-carbonester der Formel I erhalten werden.

| Nr. | $R^1$ | $R^2$ | Fp [°C] |
|---|---|---|---|
| 1 | $CH_3$ | $CH_3$ | 113-114 |
| 2 | $CH_3$ | $C_2H_5$ | 94- 99 |
| 3 | $CH_3$ | n-$C_3H_7$ | 152-155 |
| 4 | $CH_3$ | i-$C_3H_7$ | 122-124 |
| 5 | $CH_3$ | n-$C_4H_9$ | 117-119 |
| 6 | $CH_3$ | s-$C_4H_9$ | |
| 7 | $CH_3$ | t-$C_4H_9$ | |
| 8 | $CH_3$ | n-$C_5H_{11}$ | |
| 9 | $CH_3$ | i-$C_5H_{11}$ | |
| 10 | $CH_3$ | Cyclohexyl | 108-144 |
| 11 | $CH_3$ | Allyl | |
| 12 | $CH_3$ | Propargyl | |
| 13 | $CH_3$ | Phenyl | |

| Nr. | $R^1$ | $R^2$ | Fp [°C] |
|---|---|---|---|
| 14 | $CH_3$ | 4-Chlorphenyl | |
| 15 | $CH_3$ | 3-Chlorphenyl | |
| 16 | $CH_3$ | $CH_3OCH_2CH_2$ | |
| 17 | $CH_3$ | $CH_3SCH_2CH_2$ | |
| 18 | $CH_3$ | $ClCH_2CH_2$ | |
| 19 | $CH_3$ | $CH_3CH(Cl)CH_2$ | |
| 20 | $CH_3$ | $(CH_3)_2NCH_2CH_2$ | |
| 21 | H | $CH_3$ | |
| 22 | H | $C_2H_5$ | |
| 23 | H | $n\text{-}C_3H_7$ | |
| 24 | H | $i\text{-}C_3H_7$ | |
| 25 | H | $n\text{-}C_4H_9$ | |
| 26 | H | $i\text{-}C_4H_9$ | |
| 27 | $C_2H_5$ | $CH_3$ | |
| 28 | $C_2H_5$ | $C_2H_5$ | |
| 29 | $C_2H_5$ | $n\text{-}C_3H_7$ | |
| 30 | $C_2H_5$ | $i\text{-}C_3H_7$ | |
| 31 | $C_2H_5$ | $n\text{-}C_4H_9$ | |
| 32 | $n\text{-}C_3H_7$ | $CH_3$ | |
| 33 | $n\text{-}C_3H_7$ | $C_2H_5$ | |
| 34 | $n\text{-}C_3H_7$ | Cyclohexyl | |
| 35 | $i\text{-}C_3H_7$ | $CH_3$ | |
| 36 | $i\text{-}C_3H_7$ | $C_2H_5$ | |
| 37 | $i\text{-}C_3H_7$ | $n\text{-}C_3H_7$ | |
| 38 | $i\text{-}C_3H_7$ | $i\text{-}C_3H_7$ | |
| 39 | $n\text{-}C_4H_9$ | $CH_3$ | |
| 40 | $n\text{-}C_4H_9$ | $n\text{-}C_3H_7$ | |
| 41 | $n\text{-}C_4H_9$ | $C_2H_5$ | |
| 42 | Cyclohexyl | $CH_3$ | |
| 43 | Cyclohexyl | $C_2H_5$ | |
| 44 | Phenyl | $CH_3$ | |
| 45 | Phenyl | $C_2H_5$ | |
| 46 | Phenyl | $i\text{-}C_3H_7$ | |
| 47 | Phenyl | $n\text{-}C_3H_7$ | |
| 48 | 4-Chlorphenyl | $CH_3$ | |
| 49 | 3-Chlorphenyl | $CH_3$ | |
| 50 | 4-Fluorphenyl | $CH_3$ | |
| 51 | 4-Isopropyl-phenyl | $CH_3$ | |
| 52 | $CH_3$ | 2-Phenyl-ethyl | 153-157 |
| 53 | $i\text{-}C_3H_7$ | 2-Phenyl-ethyl | |

| Nr. | $R^1$ | $R^2$ | Fp [°C] |
|---|---|---|---|
| 54 | $i-C_3H_7$ | Cyclohexyl | 114-118 |
| 55 | $n-C_4H_9$ | $n-C_4H_9$ | viskos |
| 56 | $i-C_4H_9$ | $n-C_3H_7$ | |
| 57 | $n-C_3H_7$ | $n-C_4H_9$ | |

Die Thiophen-carbonester der Formel I können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, z.B. Methanol, Ethanol, Propanol, Butanol, Chloroform, Tetrachlorkohlenstoff, Cyclohexanol, Cyclohexanon, Chlorbenzol, Isophoron, stark polare Lösungsmittel, wie z.B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulvern, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole,

Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an festen Trägerstoffen hergestellt werden. Feste Trägerstoffe sind Mineralerden wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten zwischen 0,1 und 95 Gewichtsprozent, vorzugsweise zwischen 0,5 und 90 Gewichtsprozent, Wirkstoff.

Beispiele für Formulierungen sind:

I. Man vermischt 90 Gewichtsteile der Verbindung Nr. 1 mit 10 Gewichtsteilen N-Methyl-alpha-pyrrolidon und erhält eine Lösung, die zur Anwendung in Form kleinster Tropfen geeignet ist.

II. 20 Gewichtsteile der Verbindung Nr. 3 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-mono-ethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gewichtsprozent des Wirkstoffs enthält.

III. 20 Gewichtsteile der Verbindung Nr. 2 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

IV. 20 Gewichtsteile der Verbindung Nr. 4 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

V. 20 Gewichtsteile der Verbindung Nr. 10 werden mit 3 Gewichtsteilen des Natriumsalzes der Diisobutylnaphthalin-alpha-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gewichtsprozent des Wirkstoffs enthält.

VI. 3 Gewichtsteile der Verbindung Nr. 1 werden mit 97 Gewichtsteilen feinteiligem Kaolin vermischt. Man erhält auf diese Weise ein Stäubemittel, das 3 Gewichtsprozent des Wirkstoffs enthält.

VII. 30 Gewichtsteile der Verbindung Nr. 2 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Teile der Verbindung Nr. 1 werden mit 2 Teilen Calciumsalz der Dodecylbenzolsulfonsäure, 8 Teilen Fettalkohol-polyglykolether, 2 Teilen Natriumsalz eines Phenol-Harnstoff-Formaldehyd-Kondensates und 68 Teilen eines paraffinischen Mineralöls innig vermischt. Man erhält eine stabile ölige Dispersion.

Die Applikation der Wirkstoffe bzw. der Mittel kann im Vorauflaufverfahren oder im Nachauflaufverfahren erfolgen. Sind die Wirkstoffe für die Kulturpflanzen weniger verträglich, so können auch Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Bodenart, Jahreszeit, Zielpflanzen und Wachstumsstadium 0,1 bis 5 kg/ha und mehr, vorzugsweise 0,5 bis 3 kg/ha.

Die herbizide Wirkung von Verbindungen der Formel I wird durch Gewächshausversuche gezeigt:

Als Kulturgefäße dienen Plastikblumentöpfe mit 300 $cm^3$ Inhalt und lehmigem Sand mit etwa 1,5 % Humus als Substrat.

Die Samen der Testpflanzen werden nach Arten getrennt flach eingesät. Unmittelbar danach werden die Wirkstoffe bei Vorauflaufbehandlung auf die Erdoberfläche aufgebracht. Sie werden hierzu in Wasser als Verteilungsmittel suspendiert oder emulgiert und mittels fein verteilender Düsen gespritzt. Die Aufwandmengen betragen dabei 3,0 und 1,0 kg Wirkstoff/ha. Nach dem Aufbringen der Mittel werden die Gefäße leicht beregnet, um Keimung und Wachstum in Gang zu bringen. Danach werden die Gefäße mit durchsichtigen Plastikhauben abgedeckt, bis die Pflanzen angewachsen sind. Diese Abdeckung bewirkt ein gleichmäßiges Keimen der Testpflanzen, sofern dies nicht durch die Wirkstoffe beeinträchtigt wird.

Für die Nachauflaufbehandlung werden die Testpflanzen je nach Wuchsform erst bis zu einer Wuchshöhe von 3 bis 15 cm angezogen und danach behandelt. Die für die Nachauflaufanwendung eingesetzten Soja- und Reispflanzen werden in einem mit Torfmull (peat) angereicherten Substrat angezogen, um ein günstigeres Wachstum zu gewährleisten. Zur Nachauflaufbehandlung werden entweder direkt gesäte und in den gleichen Gefäßen aufgewachsene Pflanzen ausgewählt, oder aber sie werden erst als Keimpflanzen getrennt angezogen und einige Tage vor der Behandlung in die Versuchsgefäße verpflanzt. Eine Abdeckung unterbleibt bei der Nachauflaufbehandlung. Die Aufwandmenge beträgt beispielsweise 1,0 kg Wirkstoff/ha.

Die Versuchsgefäße werden im Gewächshaus aufgestellt, wobei für wärmeliebende Arten wärmere Bereiche (20 bis 30°C) und für solche gemäßigter

Klimate 15 bis 25°C bevorzugt werden. Die Versuchsperiode erstreckt sich über 2 bis 4 Wochen. Während dieser Zeit werden die Pflanzen gepflegt, und ihre Reaktion auf die einzelnen Behandlungen wird ausgewertet. Bewertet wird nach einer Skala von 0 bis 100. Dabei bedeutet 0 keine Schädigung oder normaler Auflauf und 100 kein Aufgang der Pflanzen bzw. völlige Zerstörung zumindest der oberirdischen Teile.

Die Testpflanzen setzen sich aus folgenden Arten zusammen: Arachys hypogaea (Erdnüsse), Avena fatua (Flughafer), Chenopodium album (Weißer Gänsefuß), Galium aparine (Klettenlabkraut), Gossypium hirsutum (Baumwolle), Lamium amplexicaule (stengelumfassende Taubnessel), Mercurialis annua (einjähriges Bingelkraut), Oryza sativa (Reis), Sinapis alba (weißer Senf), Solanum nigrum (schwarzer Nachtschatten), Triticum aestivum (Weizen) und Veronica spp. (Ehrenpreisarten).

Bei Vorauflaufanwendung zeigen beispielsweise die Verbindungen Nr. 1, 2, 3 und 10 eine beachtliche herbizide Aktivität, insbesondere gegen Sinapis alba. Ferner bekämpft Verbindung Nr. 4 bei dieser Anwendungsmethode unerwünschte breitblättrige Pflanzen selektiv in Weizen.

Bei Nachauflaufanwendung bekämpft beispielsweise Verbindung Nr. 1 eine ganze Reihe unerwünschter breitblättriger Pflanzen.

In Anbetracht der Verträglichkeit und der Vielseitigkeit der Applikationsmethoden, können die erfindungsgemäßen Verbindungen noch in einer weiteren Zahl von Kulturpflanzen zur Beseitigung unerwünschter Pflanzen eingesetzt werden.

In Betracht kommen beispielsweise folgende Kulturen:

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Avena sativa | Hafer |
| Beta vulgaris spp. altissima | Zuckerrübe |
| Beta vulgaris spp. rapa | Futterrübe |
| Beta vulgaris spp. esculenta | Rote Rübe |
| Brassica napus var. napus | Raps |
| Brassica napus var. napobrassica | Kohlrübe |
| Brassica napus var. rapa | Weiße Rübe |

| Botanischer Name | Deutscher Name |
|---|---|
| Brassica rapa var. silvestris | Rübsen |
| Camellia sinensis | Teestrauch |
| Carthamus tinctorius | Saflor - Färberdistel |
| Carya illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cucumis melo | Melone |
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glycine max | Sojabohne |
| Gossypium hirsutum (Gossypium arboreum Gossypium herbaceum Gossypium vitifolium) | Baumwolle |
| Helianthus annuus | Sonnenblume |
| Helianthus tuberosus | Topinambur |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lactua sativa | Kopfsalat |
| Lens culinaris | Linse |
| Linum usitatissimum | Faserlein |
| Lycopersicon lycopersicum | Tomate |
| Malus spp. | Apfel |
| Manihot esculenta | Maniok |
| Medicago sativa | Luzerne |
| Mentha piperita | Pfefferminze |
| Musa spp. | Obst- u. Mehlbanane |
| Nicotiana tabacum (N. rustica) | Tabak |
| Olea europaea | Ölbaum |
| Oryza sativa | Reis |

| Botanischer Name | Deutscher Name |
|---|---|
| Panicum miliaceum | Rispenhirse |
| Phaseolus lunatus | Mondbohne |
| Phaseolus mungo | Erdbohne |
| Phaseolus vulgaris | Buschbohnen |
| Pennisetum glaucum | Perl- oder Rohrkolbenhirse |
| Petroselinum crispum spp. tuberosum | Wurzelpetersilie |
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sesamum indicum | Sesam |
| Solanum tuberosum | Kartoffel |
| Sorghum bicolor (s. vulgare) | Mohrenhirse |
| Sorghum dochna | Zuckerhirse |
| Spinacia oleracea | Spinat |
| Theobroma cacao | Kakaobaum |
| Trifolium pratense | Rotklee |
| Triticum aestivum | Weizen |
| Vaccinium carymbosum | Kulturheidelbeere |
| Vaccinium vitis-idaea | Preißelbeere |
| Vicia faba | Pferdebohnen |
| Vigna sinensis (V. unguiculata) | Kuhbohne |
| Zea mays | Mais |

Zur Verbreiterung des Wirkungsspektrums und zur Erzielung synergistischer Effekte können die Thiophen-carbonester der Formel I mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate und andere in Betracht.

Außerdem kann es von Nutzen sein, die Thiophen-carbonester der Formel I bzw. sie enthaltende herbizide Mittel allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- oder Spurenelementmängeln eingesetzt werden. Es können auch nichtphytotoxische Öle und Ölkonzentrate zugesetzt werden.

Patentansprüche

1. Thiophen-carbonester der Formel

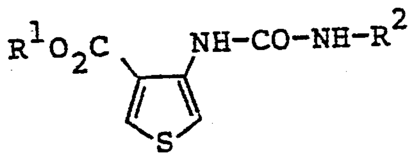

(I),

in der

$R^1$ Wasserstoff, $C_1$-$C_{10}$-Alkyl, $C_2$-$C_{10}$-Alkenyl, $C_2$-$C_{10}$-Alkinyl, $C_1$-$C_{10}$-Halogenalkyl, $C_2$-$C_{10}$-Alkoxyalkyl, $C_2$-$C_{10}$-Alkylthioalkyl, $C_3$-$C_7$-Cycloalkyl, gegebenenfalls durch Halogen oder $C_1$-$C_4$-Alkyl substituiertes Phenyl oder gegebenenfalls durch Halogen substituiertes Benzyl und

$R^2$ $C_1$-$C_{10}$-Alkyl, $C_2$-$C_{10}$-Alkenyl, $C_2$-$C_{10}$-Alkinyl, gegebenenfalls durch Halogen substituiertes $C_7$-$C_{10}$-Phenylalkyl, $C_1$-$C_{10}$-Halogenalkyl, $C_2$-$C_{10}$-Alkoxyalkyl, $C_2$-$C_{10}$-Alkylthioalkyl, durch Alkylamino oder Dialkylamino mit 1 bis 4 C-Atomen in einer Alkylgruppe substituiertes $C_1$-$C_{10}$-Alkyl, $C_3$-$C_7$-Cycloalkyl oder gegebenenfalls durch Halogen oder $C_1$-$C_4$-Alkyl substituiertes Phenyl bedeuten.

2. Thiophen-carbonester der Formel I gemäß Anspruch 1, <u>dadurch gekennzeichnet</u>, daß

$R^1$ Wasserstoff, $C_1$-$C_4$-Alkyl, $C_3$-$C_4$-Alkenyl, $C_3$-$C_4$-Alkinyl, $C_1$-$C_4$-Halogenalkyl, $C_2$-$C_4$-Alkoxyalkyl, $C_2$-$C_4$-Alkylthioalkyl, $C_5$-$C_6$-Cycloalkyl und

$R^2$ $C_1$-$C_4$-Alkyl, $C_3$-$C_4$-Alkenyl, $C_3$-$C_4$-Alkinyl, $C_8$-$C_9$-Phenylalkyl, $C_1$-$C_4$-Halogenalkyl, $C_2$-$C_4$-Alkoxyalkyl, $C_2$-$C_4$-Alkylthioalkyl, durch Alkylamino oder Dialkylamino mit 1 bis 4 C-Atomen substituiertes $C_1$-$C_4$-Alkyl oder $C_5$-$C_6$-Cycloalkyl bedeuten.

3. Thiophen-carbonester der Formel I gemäß Anspruch 1, <u>dadurch gekennzeichnet</u>, daß $R^1$ $C_1$-$C_4$-Alkyl und $R^2$ $C_1$-$C_4$-Alkyl oder $C_5$-$C_6$-Cycloalkyl bedeuten.

4. Thiophen-carbonester der Formel I gemäß Anspruch 3, <u>dadurch gekennzeichnet</u>, daß $R^1$ Methyl bedeutet.

5. Verfahren zur Herstellung von Thiophen-carbonestern der Formel I gemäß Anspruch 1, <u>dadurch gekennzeichnet</u>, daß man

a) einen Dihydrothiophen-carbonester der Formel

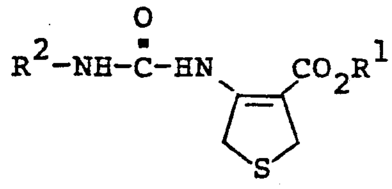

(II),

in der
$R^1$ und $R^2$ die im Anspruch 1 genannten Bedeutungen haben,

mit einem Dehydrierungsmittel oder

b) eine Aminoverbindung der Formel

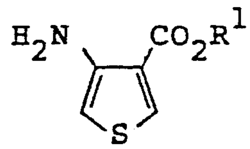

(III),

in der
$R^1$ die im Anspruch 1 genannten Bedeutungen hat, oder Salze dieser Aminoverbindung mit einem Isocyanat der Formel

$R^2$-NCO (IV),

in der $R^2$ die im Anspruch 1 genannten Bedeutungen hat, gegebenenfalls in Gegenwart einer Base in einem inerten Lösungsmittel umsetzt.

6. Herbizid, enthaltend einen Thiophen-carbonester der Formel I gemäß Anspruch 1.

7. Herbizid, enthaltend inerte Zusatzstoffe und einen Thiophen-carbonester der Formel I gemäß Anspruch 1.

8. Herbizid nach Anspruch 6, dadurch gekennzeichnet, daß es einen Thiophen-carbonester der Formel I enthält, wobei $R^1$ $C_1$-$C_4$-Alkyl und $R^2$ $C_1$-$C_4$-Alkyl oder $C_5$-$C_6$-Cycloalkyl bedeuten.

9. Verfahren zur Bekämpfung unerwünschten Pflanzenwuchses, dadurch gekennzeichnet, daß man die unerwünschten Pflanzen und/oder die von unerwünschtem Pflanzenwuchs freizuhaltenden Flächen mit einer herbizid wirksamen Menge eines Thiophen-carbonesters der Formel I gemäß Anspruch 1 behandelt.

Europäisches Patentamt

**EUROPÄISCHER RECHERCHENBERICHT**

0116932
Nummer der Anmeldung

EP 84 10 1466

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| Y | DE-A-2 040 579 (MAY & BAKER) * Ansprüche * --- | 1-9 | C 07 D 333/38<br>A 01 N 47/36 |
| Y | DE-A-2 122 636 (ESSO) * Seiten 3,4; Ansprüche * --- | 1-9 | |
| Y | US-A-3 931 204 (P. CROISIER) * Spalte 8, Zeilen 50-60; Spalte 12, Zeilen 15-23 * --- | 1,5 | |
| A | US-A-2 453 564 (B.R. BAKER) * Ansprüche * --- | 1 | |
| P,Y | EP-A-0 090 309 (BASF) * Ansprüche * ----- | 1-9 | |
| | | | RECHERCHIERTE SACHGEBIETE (Int. Cl. 3) |
| | | | C 07 D 333/00<br>A 01 N 47/00<br>A 01 N 43/00 |

Der vorliegende Recherchenbericht wurde für alle Patentansprüche erstellt.

| Recherchenort | Abschlußdatum der Recherche | Prüfer |
|---|---|---|
| DEN HAAG | 22-05-1984 | CHOULY J. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82